# EUROPEAN PATENT SPECIFICATION

(11) **EP 4 547 325 B1**
(45) Date of publication and mention of the grant of the patent: **02.12.2026**
(21) Application number: 23737928.4
(22) Date of filing: 29.06.2023
(51) Int. Cl.: A61N 1/39

(54) **DEFIBRILLATOR ELECTRODE ILLUMINATION FOR GUIDANCE**
DEFIBRILLATORELEKTRODENBELEUCHTUNG ZUR FÜHRUNG
ÉCLAIRAGE D'ÉLECTRODE DE DÉFIBRILLATEUR POUR GUIDAGE

(30) Priority: 30.06.2022 US 202263357217 P
(43) Date of publication of application: 07.05.2025
(73) Proprietor: Everest Acquisition Entity, LLC, Wilmington, DE 19805 (US)
(72) Inventor: FIEBIG, Mark, 5656 AG Eindhoven (NL); RICHARD, Christian James, 5656AG Eindhoven (NL)
(74) Representative: Modiano, Gabriella Diana
(86) International application number: PCT/EP2023/067752
(87) International publication number: WO 2024/003204

(56) References cited:
- CN-A- 114 364 324
- US-A1- 2017 056 650
- US-B2- 11 058 866

## Description

### FIELD OF THE INVENTION

The present disclosure generally relates to a guidance for an operational user of a defibrillator (e.g., an Automated External Defibrillator (AEDs), an Advanced Life Support (ALS) defibrillator and a Basic Life Support (BLS) defibrillator). The present disclosure specifically relates to a visual guidance for the operational user of a defibrillator.

### BACKGROUND OF THE INVENTION

Automated external defibrillators (AEDs) and monitor/defibrillator systems commonly use disposable electrodes to deliver a therapy shock to patients. The current state of the art for disposable defibrillator electrodes includes foam or paper pads with a flexible metallic core and conductive gel that adheres the electrodes to the patient during therapy. The electrodes contain printed imagery and/or graphic labels that indicate to the operational user of the defibrillator how the electrodes are to be placed on the patient and may further contain cautions and warnings related to such use. The electrodes also include a split electrical cable that connects to the defibrillator device. More particularly, this cable delivers electrical resistance and electrocardiogram (ECG) data from the electrodes to the defibrillator and provides electrical energy from the defibrillator to the electrodes to provide a defibrillation shock.

Several of the most significant problems associated with AEDs is the inexperience of the user, the rapid degradation of the patient during a cardiac arrest, and the safety risks associated with providing a high-voltage shock in a public setting. These challenges are widely recognized and generally common to all AEDs. The devices are intended to be used by untrained and minimally trained users, so the usability of the AED design needs to encourage the users to identify and perform the steps of the therapy workflow as accurately and quickly as possible.

The effectiveness of automated external defibrillators, which are used by untrained, minimally trained, or trained operational users, is highly dependent on the speed with which the user can find and deploy the electrodes onto the patient's chest. Once the electrodes are on the patient's chest, the defibrillator analyzes the heart rhythm and to make a shock/no-shock decision, and provides audio and/or visual indications to not touch the patient. If a shock is deemed necessary, the defibrillator prompts the user to press the Shock button or initiates the shock automatically. There are safety risks to the user(s) and bystanders (e.g., unintended electrical shock) and to the patient (e.g., delay in defibrillation therapy) if anyone is touching the patient during the shock/no-shock analysis period or while the shock is being delivered.

For defibrillators currently known or available in the market, electrodes and cables have minimal information to guide the user, basically limited to only a pads placement diagram, printed symbols, and printed warnings and cautions, with no dynamic indicators to aid the user. U.S. Patent No. 11,058,866 B2 to Andrews teaches graphically responsive labels on the electrodes, such that electrochromic layers show specific printed labels of "Perform CPR" and "Do Not Touch Patient" on the electrodes at appropriate times during the rescue. Although *Andrews* provides dynamic point-of-use information related solely to the therapy shock, *Andrews* relies on the user reading the printed information on the electrodes and does not teach an illumination of the electrodes as a point-of-use visual indication of a functioning status of the distribution unit. *Andrews* also does not provide the user dynamic information during the defibrillator turn-on and pads application tasks.

Document CN 114 364 324 A entitled "System And Method For Medical Ultrasound With Monitoring Pad" teaches LEDs, in an ultrasound probe and cable, may be used to signal to an operator the status of the ultrasound system.

### SUMMARY OF THE INVENTION

The invention is defined by the independent claims. The present disclosure is directed to a defibrillation unit employing a defibrillator, electrode(s) and a cable coupling the electrode(s) to the defibrillator, whereby light indicators provided in the electrode(s) and cable are controlled by logic in a light indicator controller within the defibrillator, the electrode, or an additional device. More particularly, as the defibrillator operates through therapy workflow stages, the light indicator controller controls an illumination of the electrode(s) and cable via the light indicators to provide a point-of-use visual indication of the functioning state of the defibrillation unit, and may synchronize such control with voice prompts and/or visual displays by the defibrillator to provide instructions to the user and warnings to any bystanders.

The present disclosure can be exemplarily embodied as:
(1) a defibrillation unit (e.g., may be incorporated in automated external defibrillators and other monitoring/defibrillation systems as known in the art of the present disclosure or hereinafter conceived);
(2) a light indicator controller for controlling an indication of a functioning status of a defibrillation unit; and
(3) a method executable by the light indicator controller for controlling an indication of a functioning status of a defibrillation unit.

Various defibrillation unit embodiments of the present disclosure encompass a defibrillator, an electrode and a cable for coupling the electrode to the defibrillator. The electrode includes an electrode light indicator, and the cable includes a cable light indicator. The defibrillation unit embodiments further encompass a light indicator controller for controlling an indication of a functioning status of the defibrillation unit. To this end, the light indicator controller is configured to ascertain an operational mode of the defibrillator and (2) control an illumination of the electrode light indicator and the cable light indicator based on the operational mode of the defibrillator.

Various light indicator controller embodiments of the present disclosure encompass a non-transitory machine-readable storage medium encoded with instructions for execution by one or more processors for controlling an indication of a functioning status of an embodiment of the defibrillation unit of the present disclosure. The non-transitory machine-readable storage medium includes the instructions to (1) ascertain an operational mode of the defibrillator and (2) control an illumination of the electrode light indicator and the cable light indicator based on the operational mode of the defibrillator.

Various methods embodiments of the present disclosure executable by the light indicator controller for controlling an indication of a functioning status of an embodiment of the defibrillation unit of the present disclosure involves the light indicator controller (1) ascertaining an operational mode of the defibrillator, and (2) controlling an illumination of the electrode light indicator and the cable light indicator based on the operational mode of the defibrillator.

The foregoing exemplary embodiments and other embodiments of the present disclosure as well as various structures and advantages of the present disclosure will become further apparent to one having ordinary skill in the art from the following detailed description of various exemplary embodiments of the present disclosure read in conjunction with the accompanying drawings and claims. The detailed description and drawings are merely illustrative of the present disclosure rather than limiting, the scope of the present disclosure being defined by the appended claims.

### BRIEF DESCRIPTION OF THE DRAWINGS

The present disclosure will present in detail the following description of exemplary embodiments with reference to the following figures wherein:
FIG. 1 illustrates an exemplary embodiment of a defibrillation unit in accordance with the present disclosure;
FIG. 2 illustrates an exemplary embodiment of the defibrillator illustrated in FIG. 1 in accordance with the present disclosure;
FIG. 3 illustrates an exemplary embodiment of the electrode and the defibrillator illustrated in FIG. 1 in accordance with the present disclosure;
FIG. 4 illustrates an exemplary embodiment of a medical tablet in accordance with the present disclosure;
FIG. 5 illustrates an exemplary embodiment of a flowchart representative of point-of-use light indication method in accordance with the present disclosure;
FIG. 6 illustrates an exemplary embodiment of the light indicator controller of FIG. 1 in accordance with the present disclosure;
FIG. 7 illustrates an exemplary embodiment of the flowchart in FIG. 5 in accordance with the present disclosure;
FIGS. 8A-8D illustrate an exemplary embodiment illumination of electrodes and a cable during a shock therapy procedure in accordance with the present disclosure; and
FIG. 9 illustrates an exemplary embodiment of a light indicator controller of FIG. 6 in accordance with the present disclosure.

### DETAILED DESCRIPTION OF THE PREFERRED EMBODIMENTS

Exemplary embodiments of a defibrillation unit in accordance with the present disclosure can be applicable to any type of medical procedure involving a therapy shock being applied to a patient's heart by a defibrillator via electrodes coupled to the defibrillator by a cable.

For purposes of describing and claiming the present disclosure, the term "defibrillator" broadly encompasses any electronic device, as known in the art of the present disclosure or hereinafter conceived, for providing an electric pulse or shock to a patient's heart in an attempt to restore a normal functioning of the patient's heart; the term "electrode" broadly encompasses any conductive tool, as known in the art of the present disclosure or hereinafter conceived, for conforming to a patient's body to deliver an electric pulse or a shock from a defibrillator to the patient's heart; and the term "cable" broadly encompasses any conductive connector, as known in the art of the present disclosure or hereinafter conceived, for coupling electrode(s) to a defibrillator.

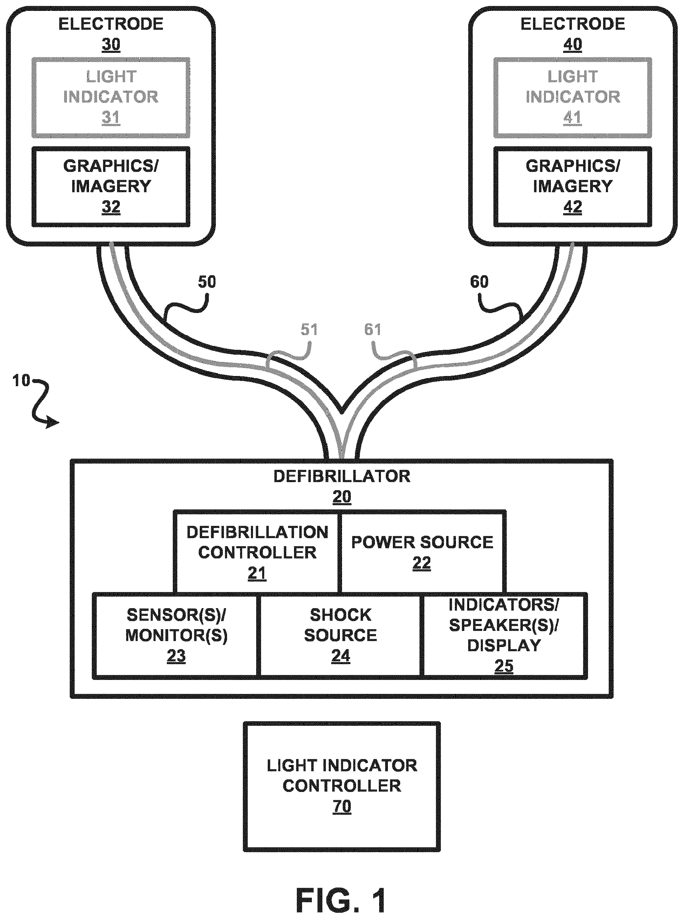

To facilitate an understanding of the present disclosure, the following description of FIGS. 1-4 teaches exemplary embodiments of a defibrillation units and various components thereof in accordance with the present disclosure. From the description of FIGS. 1-4, those having ordinary skill in the art of the present disclosure will appreciate how to apply the present disclosure to make and use additional embodiments of defibrillation units and various components thereof in accordance with the present disclosure.

Referring to FIG. 1, an exemplary defibrillation unit 10 of the present disclosure employs a defibrillator 20, a pair of electrodes 30 and 40, a split cable having a cable 50 and a cable 60 with cable 50 coupling defibrillator 20 to electrode 30 and cable 60 coupling defibrillator 40 to defibrillator 20.

Exemplary defibrillator 20 includes a defibrillation controller 21, a power source 22 (e.g., a battery), sensor(s)/monitor(s) 23, a shock source 24, and indicator(s)/speaker(s)/display 25 as known in the art of the present disclosure. In training embodiments of the present disclosure, a defibrillator would exclude shock source 24.

Exemplary defibrillation controller 21 is programmed with various algorithms for controlling a delivery of a therapy shock to a patient's heart when powered by the power source 22. Nonlimiting examples of such algorithms include algorithms (1) for detecting and identifying non-shockable heart rhythms and/or shockable heart rhythms as sensed and monitored by sensors/monitors 23 via electrodes 30 and 40, (2) for managing a charging of shock source 24 prior to the therapy shock and managing a discharging of shock source 24 during a therapy shock to the patient's heart via electrodes 30 and 40, and (3) for utilizing indicator(s)/speaker(s)/display 25 to provide instructions/warnings to the user and any bystanders prior to and/or during the therapy shock.

In practice, in accordance with certain exemplary embodiments of the present disclosure, electrodes 30 and 40 can further include graphics/imagery 32 as known in the art of the present disclosure or hereinafter conceived for providing instructions to a user of defibrillation unit 10. Also in practice, in accordance with certain exemplary embodiments of the present disclosure, electrodes 30 and 40 can include flat audio speakers (not shown) as known in the art of the present disclosure or hereinafter conceived for providing point-of-use audio instructions to the user of defibrillation unit 10 and/or an active flexible display (not shown) as known in the art of the present disclosure or hereinafter conceived for providing point-of-use video instructions to the user of defibrillation unit 10.

Still referring to FIG. 1, exemplary defibrillation unit 10 further employs a light indicator controller 70 of the present disclosure for controlling an electrode light indicator 31 of electrode 30, an electrode light indicator 41 of electrode 40, a cable light indicator 51 of cable 50 and a cable light indicator 60 of cable 60.

For purposes of describing and claiming the present disclosure, the term "light indicator" broadly encompasses any type of illumination source or arrangement of illumination sources, as known in the art of the present disclosure or hereinafter conceived, having one or more properties that may be varied, such as, for example, an illumination color, an illumination intensity and an illumination modulation functionality.

In one exemplary embodiment, for example, a light indicator may be a light emitting diode (LED) strategically positioned within or on an electrode or a cable for optimal visualization of an illumination of the LED as controlled by light indicator controller 70.

In a second exemplary embodiment, for example, a light indicator can be LEDs arranged as a string or in a geometrical shape and strategically positioned within or on an electrode or a cable for optimal visualization of an illumination of the LEDs as controlled by light indicator controller 70.

For exemplary embodiments having the LED positioned within an electrode or cable, the electrode or the cable can be transparent or translucent.

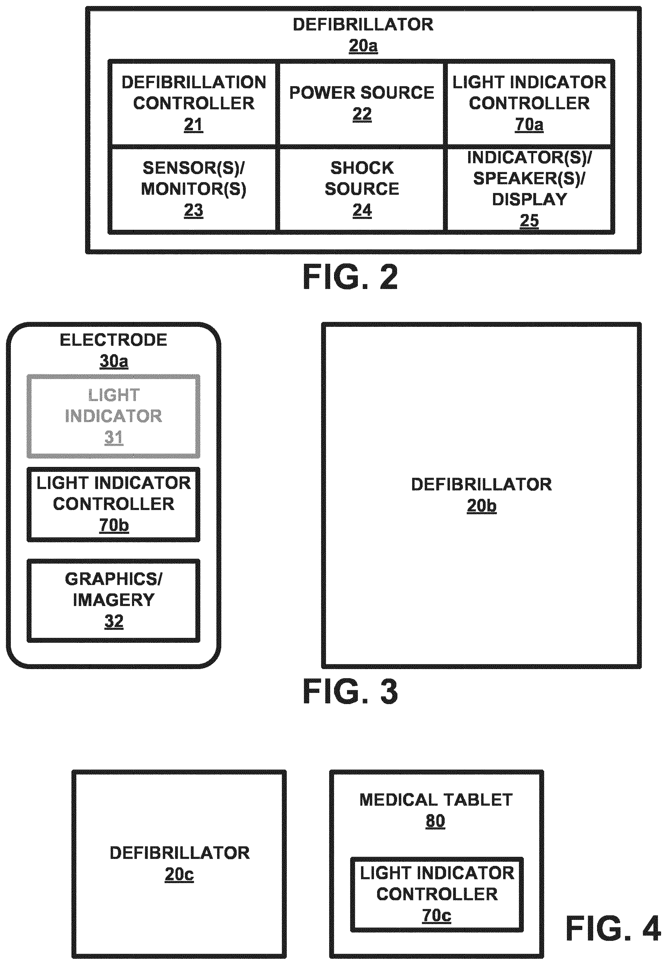

In practice, in accordance with certain exemplary embodiments of the present disclosure, a light indicator controller 70a may be installed within a defibrillator 20a as shown in FIG. 2, whereby light indicator controller 70a is either segregated from defibrillation controller 21 and powered by power source 22, or integrated into defibrillation controller 21. For either exemplary embodiment, defibrillator 20a can include an interface (not shown) as known in the art of the present disclosure for facilitating defibrillation controller 21, sensor(s)/monitors 23 and shock source 24 communicating with the electrodes relating to the sensing/monitoring the patient's heart and any therapy shock delivered to the patient's heart. Also for either exemplary embodiment, defibrillator 20a can further include an additional interface (not shown) as would be appreciated by those having ordinary skill in the art of the present disclosure for facilitating a powering of the light indicators via power source 22 as controlled by light indicator controller 70a.

Also in practice, in accordance with certain exemplary embodiments of the present disclosure, a light indicator controller 70b may be installed within an electrode 30a as shown in FIG. 3. For this exemplary embodiment, a defibrillator 20b can include an interface (not shown) as known in the art of the present disclosure for facilitating defibrillation controller 21, sensor(s)/monitors 23 and shock source 24 communicating with the electrodes relating to the sensing/monitoring the patient's heart and any therapy shock delivered to the patient's heart. Also for this exemplary embodiment, defibrillator 20b can further include additional interface (not shown) as would be appreciated by those having ordinary skill in the art of the present disclosure for facilitating a powering of the light indicators via power source 22 as controlled by light indicator controller 70b.

Additionally in practice, in accordance with certain exemplary embodiments of the present disclosure, a light indicator controller 70c may be installed within a medical tablet 80 as shown in FIG. 4. For this exemplary embodiment, a defibrillator 20c can include an interface (not shown) as known in the art of the present disclosure for facilitating defibrillation controller 21, sensor(s)/monitors 23 and shock source 24 communicating with the electrodes relating to the sensing/monitoring the patient's heart and any therapy shock delivered to the patient's heart. Also for this exemplary embodiment, defibrillator 20c can further include additional wired/wireless interface (not shown) as would be appreciated by those having ordinary skill in the art of the present disclosure for facilitating a powering of the light indicators via power source 22 as controlled by light indicator controller 70b. This exemplary embodiment is particularly useful when different types of electrodes may be coupled to defibrillator 20c.

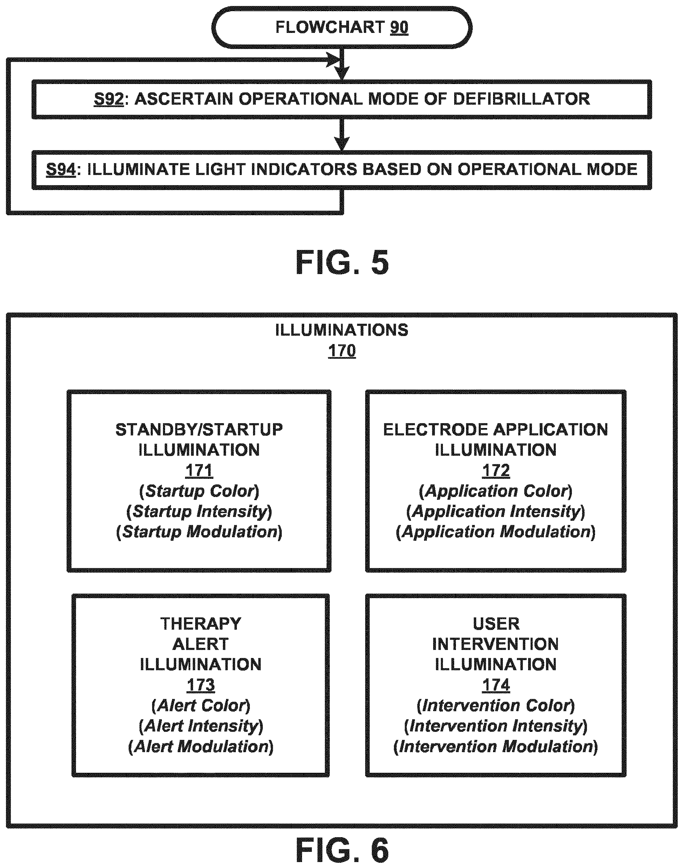

FIG. 5 illustrates a flowchart 90 representative of an exemplary point-of-use light indication method of the present disclosure that is executable by light indicator controller 70 for indicating a functional status of a defibrillation unit (e.g., defibrillation unit 10 of FIG. 1).

Referring to FIG. 5, in accordance with certain exemplary embodiments of the present disclosure, a stage S92 of flowchart 90 encompasses light indicator controller 70 ascertaining an operational mode of a defibrillator (e.g., defibrillator 20 of FIG. 1) via communication from a defibrillation controller or as integrated in the defibrillation controller. In practice, a series of operational modes of the defibrillator can depend upon the workflow of therapy being applied to a patient's heart, for example.

In one workflow exemplary embodiment, a shock therapy consists of a startup phase to an electrode application phase to a heart rhythm analysis/shock delivery phase to an intervention phase (e.g. to perform cardiopulmonary resuscitation (CPR)).

In a second workflow exemplary embodiment, a shock therapy consists of a standby/startup phase to an electrode application phase to a heart rhythm analysis/shock delivery phase to an intervention phase (e.g., CPR, ventilation, etc.).

Still referring to FIG. 5, in accordance with certain exemplary embodiments of the present disclosure, a stage S94 of flowchart 90 encompasses light indicator controller 70 controlling an illumination of the electrode light indicator(s) and the cable light indicator(s) based on the operational mode of the defibrillator queried in stage S92. In practice, the illumination of each operation mode of the defibrillator can be based on various proprieties of the electrode light indicator(s) and the cable light indicator(s). Non-limiting examples of such proprieties include color, intensity and modulation.

In one exemplary embodiment as shown in FIG. 6, for example, stage S94 has four types of illuminations 170. The first illumination type is a standby/startup illumination 171 having distinct color(s), distinct intensity(ies) and/or distinct modulation(s) representative of a deactivation (standby) and/or an activation (startup) of the defibrillator when the electrode is coupled to the defibrillator. The second illumination type is an electrode application illumination 172 having distinct color(s), distinct intensity(ies) and/or distinct modulation(s) representative of a detection by the defibrillator that the electrodes have been applied to the patient. The third illumination type is a therapy alert illumination 173 having distinct color(s), distinct intensity(ies) and/or distinct modulation(s) (e.g., color variation, intensity variation, frequency variation) representative of a heart rhythm analysis and/or a shock delivery by the defibrillator. The fourth illumination type is a user intervention illumination 174 having distinct color(s), distinct intensity(ies) and/or distinct modulation(s) representative of user intervention being monitored by the defibrillator (e.g., an ECG monitoring of a CPR or ventilation).

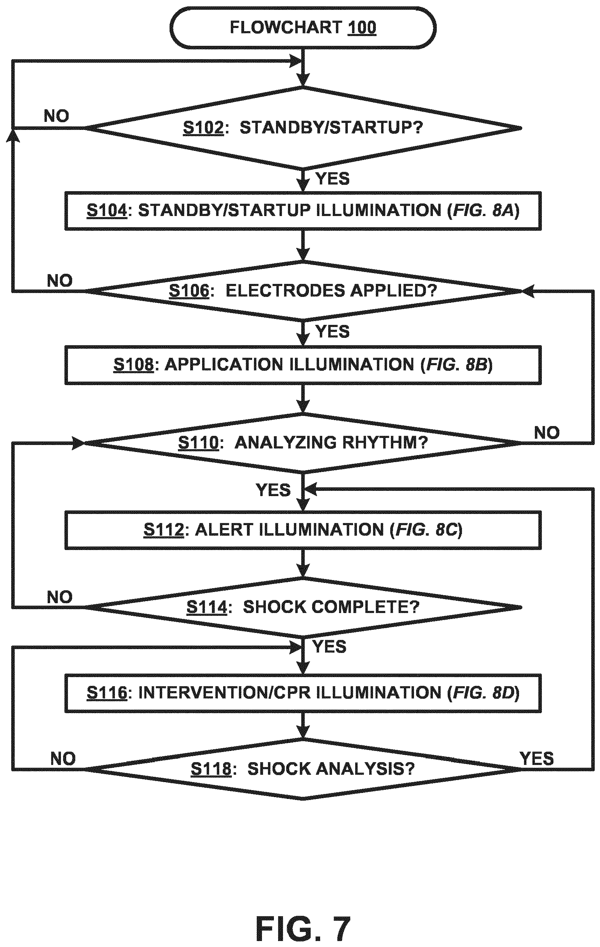

FIG. 7 illustrates a flowchart 110 representative of point-of-use light indication method of the present disclosure based on the illumination types 170 of FIG. 6.

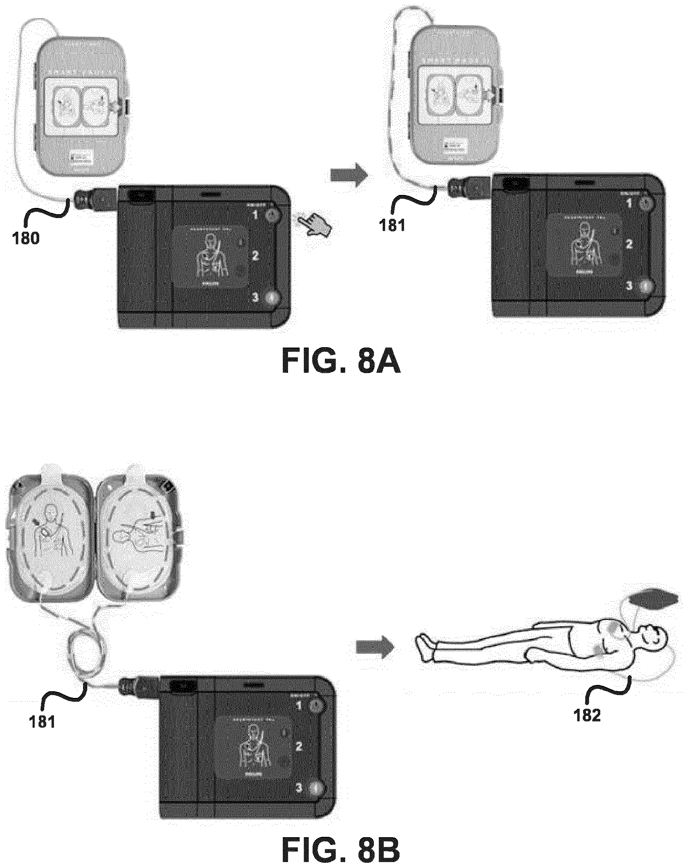

Referring to FIG. 7, in accordance with certain exemplary embodiments of the present disclosure, a stage S112 of flowchart 110 encompasses light indicator controller 70 determining if the defibrillator is in a standby mode (deactivation with electrodes coupled thereto) or a startup mode (activation with electrode coupled thereto). If the defibrillator is determined to be in a standby mode during stage S112, then the light indicator controller 70 proceeds to a stage S114 of flowchart S110 to control a standby illumination of the electrode light indicators (not shown) and the cable light indicators at a distinct color over the length of the light indicators (e.g., illumination of all LEDS throughout the light indicators at a blue color), such as, for example, a standby illumination 180 of the light indicators as shown in FIG. 8A.

Thereafter, if the defibrillator is determined to transition from the standby mode to a startup mode during stage S112, then the light indicator controller 70 returns to stage S114 of flowchart 110 to control a startup illumination of the electrode light indicators (not shown) and the cable light indicator sat a distinct color and pattern (e.g., illumination of all alternating LEDS throughout the light indicators at a blue color), such as, for example, a startup illumination 181 of the light indicators as shown in FIG. 8A. The user of the defibrillator thus has a visual cue that the defibrillator is fully activated.

An exemplary stage S116 of flowchart 110 encompasses light indicator controller 70 determining if the electrodes have been applied to the patient. If so, then the light indicator controller 70 proceeds to a stage S118 of flowchart 110 to control an application illumination of the electrode light indicators and the cable light indicators at a distinct color over the length of the light indicators (e.g., illumination of all LEDS throughout the light indicators at a green color), such as, for example, an application illumination 182 of the light indicators as shown in FIG. 8B. The user of the defibrillator thus has a visual cue that the defibrillator is ready for shock therapy.

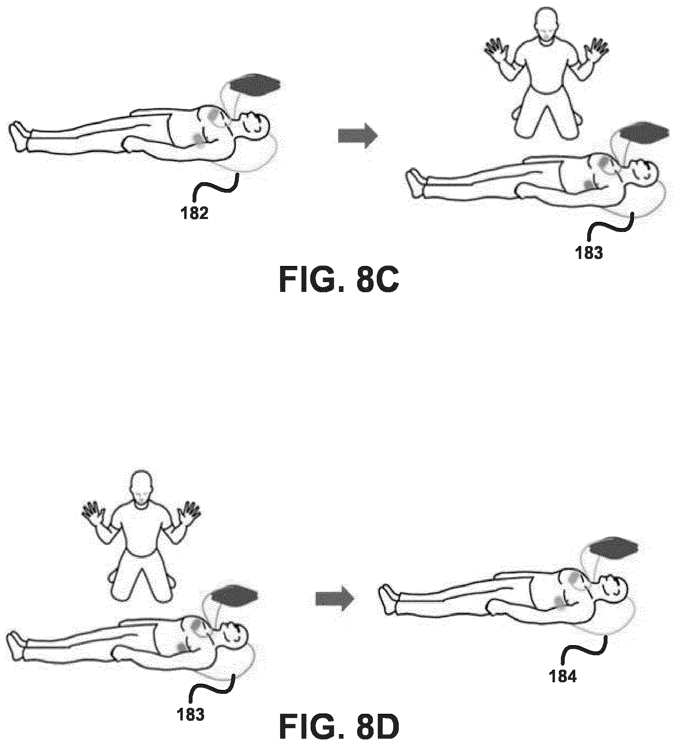

An exemplary stage S120 of flowchart 110 encompasses light indicator controller 70 determining if the defibrillation controller is executing a heart rhythm analysis. If so, then the light indicator controller 70 proceeds to a stage S122 of flowchart 110 to control a therapy alert illumination of the electrode light indicators and the cable light indicators at a distinct color over the length of the light indicators (e.g., illumination of all LEDs throughout the light indicators at a red color), such as, for example, an application illumination 183 of the light indicators as shown in FIG. 8C. During a stage S122 of flowchart 110, light indicator controller 70 determines if the defibrillation controller is executing a shock therapy while light indicator controller 70 is still controlling a therapy alert illumination of the electrode light indicators and the cable light indicators. The user of the defibrillator and bystanders thus have a visual cue to stand back from the patient as shock therapy is being administered.

Upon expiration of a safe time period after the shock delivery, light indicator controller 70 proceeds to a stage S126 of flowchart 120 to control a user intervention illumination of the electrode light indicators and the cable light indicators at a distinct color over the length of the light indicators (e.g., illumination of all LEDs throughout the light indicators at a yellow color), such as, for example, a user intervention illumination 184 of the light indicators as shown in FIG. 8D. The user of the defibrillator (and/or others) can thus have a visual cue that it is safe at this time to execute an intervention (e.g., CPR or ventilation), and can continue to do so until if and when the light indicator controller controls another therapy alert illumination of the light indicators, for example.

In practice of the user intervention illumination of the light indicators, in accordance with certain exemplary embodiments of the present disclosure, light indicator controller 70 can modulate the light indicators at a frequency guiding the intervention (e.g., modulate at desired CPR rate).

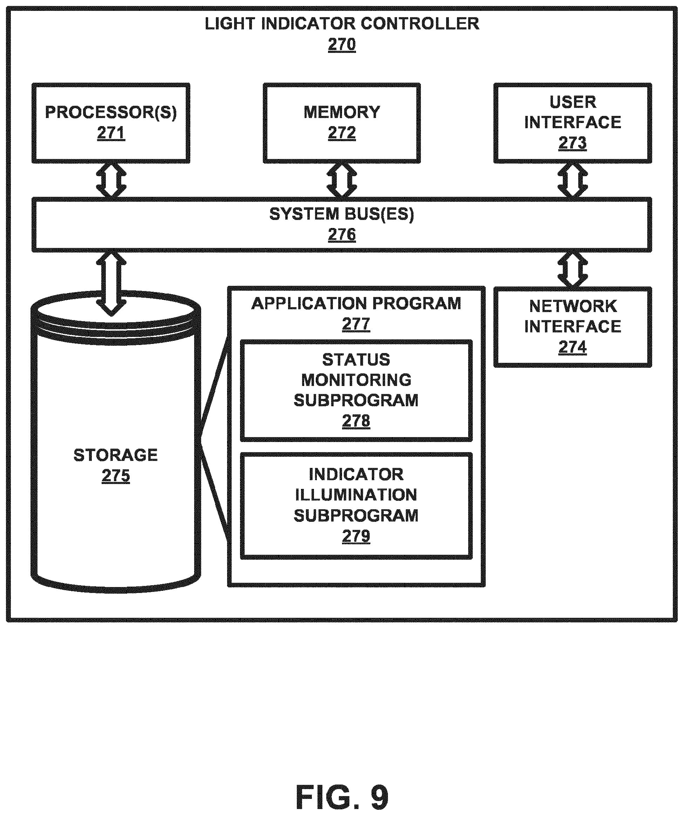

To facilitate a further understanding of the present disclosure, the following description of FIG. 9 teaches an exemplary embodiment of light indicator controller in accordance with the present disclosure. From the description of FIG. 9, those having ordinary skill in the art of the present disclosure will appreciate how to apply the present disclosure to make and use additional embodiments of a light indicator controller in accordance with the present disclosure.

Referring to FIG. 9, shown is an exemplary embodiment of light indicator controller 270 that includes one or more processor(s) 271, memory 272, a user interface 273, a network interface 274, and a storage 275 interconnected via one or more system bus(es) 276.

Each processor 271 can be any hardware device, as known in the art of the present disclosure or hereinafter conceived, capable of executing instructions stored in memory 272 or storage or otherwise processing data. In a non-limiting example, the processor(s) 271 can include a microprocessor, field programmable gate array (FPGA), application-specific integrated circuit (ASIC), or other similar devices.

The memory 272 can include various memories, as known in the art of the present disclosure or hereinafter conceived, including, but not limited to, L1, L2, or L3 cache or system memory. In a non-limiting example, the memory 272 can include static random access memory (SRAM), dynamic RAM (DRAM), flash memory, read only memory (ROM), or other similar memory devices.

The user interface 273 can include one or more devices, as known in the art of the present disclosure or hereinafter conceived, for enabling communication with a user such as an administrator, for example. In a non-limiting example, the user interface can include a command line interface or graphical user interface that can be presented to a remote terminal via the network interface 274.

The network interface 274 can include one or more devices, as known in the art of the present disclosure or hereinafter conceived, for enabling communication other components of a medical device, for example. In a non-limiting example, the network interface 274 can include a network interface card (NIC) configured to communicate according to the Ethernet protocol. Additionally, the network interface 274 can implement a TCP/IP stack for communication according to the TCP/IP protocols. Various alternative or additional hardware or configurations for the network interface 274 will be apparent to those having ordinary skill in the art.

The storage 275 can include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. In various non-limiting embodiments, the storage 275 can store instructions for execution by the processor(s) 271 or data upon with the processor(s) 271 may operate. For example, the storage 275 can store a base operating system for controlling various basic operations of the hardware.

The storage 275 can also store an application program in the form of executable software/firmware for implementing the various functions of the exemplary methods of FIGS. 5 and 7 as previously described in the present disclosure. In one exemplary embodiment as shown, for example, storage 275 can also store application program 277 including a status monitoring subprogram 278 for implementing an embodiment of stage S92 of flowchart 90 and an indicator illumination subprogram 279 for implementing an embodiment of stage S94 of flowchart 90.

Additional embodiments of the present invention include, e.g.,: illuminating the pull tabs on the electrodes, to guide the user to the tabs instead of them accidentally pulling from the bottom, for instance; illuminating the puck and cable for a CPR feedback puck placed on the patient's chest; and/or illuminating the electrode case or cartridge (not just the electrode itself), since this could draw the user to the electrodes more effectively in the first part of the workflow than illumination on the electrodes themselves.

Referring to FIGS. 1-9, those having ordinary skill in the art of the present disclosure will appreciate numerous benefits of the present disclosure including, but not limited to,

For example, typically a time from defibrillator start-up to electrode application is longest duration task in the therapy workflow, and the duration that is most impacted by the experience level of the user. One benefit of the present disclosure is a faster time from defibrillator start-up to electrode application, which is expected to improve patient outcomes.

By further example, another benefit of the present disclosure is a faster time from electrode application to heart rhythm analysis and shock delivery. The electrode and cable light indicators build user confidence during the therapy workflow and reduce error potential by rewarding actions (e.g., defibrillator activation, pads application) with visual feedback to spur on subsequent actions.

By further example, another benefit of the present disclosure is a clearer communication to the user by augmenting visual and audio indicators on the defibrillator device with dynamic visual indicators at the point of use, where the user is looking. Electrode and cable lights provide an additional secondary interface to assist users in situations where the device audio is not working or is ineffective due to environmental noise. Since defibrillators are often used in busy and loud environments, such as, e.g., airports and malls, dynamic visual indicators at the point of use represent a significant improvement over the current state of the art.

By further example, another benefit of the present disclosure is, in situations with multiple users, electrode and cable light indicators more effectively relay information to the user that is not near the defibrillator (e.g., a user delivering CPR to the patient). This reduces the safety risk of shocking users and bystanders through contact with the patient. Clear visual indication at the point of use to not touch the patient will reduce the potential for user injury due to confusion or miscoordination among users, for example.

Further, as one having ordinary skill in the art will appreciate in view of the teachings provided herein, structures, elements, components, etc. described in the present disclosure/specification and/or depicted in the Figures and/or recited in the Claims can be implemented in various combinations of hardware and software, and provide functions which can be combined in a single element or multiple elements. For example, the functions of the various structures, elements, components, etc. shown/illustrated/depicted in the Figures and/or recited in the Claims can be provided through the use of dedicated hardware as well as hardware capable of executing software in association with appropriate software for added functionality. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar function, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the present disclosure (which embodiments are intended to be illustrative and not limiting), it is noted that modifications and variations can be made by persons skilled in the art in light of the teachings provided herein, including the Figures. It is therefore to be understood that changes can be made in/to the preferred and exemplary embodiments of the present disclosure which are within the scope of the embodiments disclosed herein.

Moreover, it is contemplated that corresponding and/or related systems incorporating and/or implementing the device/system or such as may be used/implemented in/with a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure.

## Claims

1. A defibrillation unit (10), comprising:
a defibrillator (20);
an electrode (30) including an electrode light indicator (31);
a cable (50) configured to couple the electrode to the defibrillator, wherein the cable includes a cable light indicator (51); and
a light indicator controller (70) for controlling an indication of a functioning status of the defibrillation unit (10), wherein the light indicator controller (70) is configured to:
ascertain an operational mode of the defibrillator;
control an illumination of the electrode light indicator and the cable light indicator based on the operational mode of the defibrillator,
wherein
the light indicator controller is further configured to control
a standby/startup illumination of the electrode light indicator and the cable light indicator representative of at least one of a deactivation or an activation of the defibrillator when electrode is coupled to the defibrillator by cable;
an application illumination of the electrode light indicator and the cable light indicator representative of a detection, by the defibrillator, of the electrode being applied to a patient;
a therapy alert illumination of the electrode light indicator and the cable light indicator representative of at least one of a heart rhythm analysis or a shock delivery being executed by the defibrillator;
an intervention illumination of the electrode light indicator and the cable light indicator representative of an intervention monitoring being executed by the defibrillator; and,
based on the operational mode of the defibrillator, an illumination of at least one of pull tabs on the electrodes, a CPR feedback puck, or an electrode case or cartridge.

2. A light indicator controller (70) for controlling an indication of a functioning status of a defibrillation unit (10) including a defibrillator (20), an electrode (30) having an electrode light indicator (31) and a cable (50) having a cable light indicator (51), the light indicator controller (70) comprising:
a non-transitory machine-readable storage medium encoded with instructions for execution by at least one processor, the non-transitory machine-readable storage medium including the instructions to:
ascertain the functioning status of a defibrillator;
control an illumination of the electrode light indicator and the cable light indicator based on the operational mode of the defibrillator,
wherein the instructions further include to:
control a standby/startup illumination of the electrode light indicator and the cable light indicator representative of at least one of a deactivation or an activation of the defibrillator when electrode is coupled to the defibrillator by cable;.
control an application illumination of the electrode light indicator and the cable light indicator representative of a detection, by the defibrillator, of the electrode being applied to a patient;
control a therapy alert illumination of the electrode light indicator and the cable light indicator representative of at least one of a heart rhythm analysis or a shock delivery being executed by the defibrillator;
control an intervention illumination of the electrode light indicator and the cable light indicator representative of an intervention monitoring being executed by the defibrillator; and
control, based on the operational mode of the defibrillator, an illumination of at least one of pull tabs on the electrodes, a CPR feedback puck, or an electrode case or cartridge.

3. A method (110) executable by a light indicator controller (70) for controlling an indication of a functioning status of a defibrillation unit (10) including a defibrillator (20), an electrode (30) having an electrode light indicator (31) and a cable (50) having a cable light indicator (51), the method comprising:
ascertaining, by the light indicator controller, an operational mode of the defibrillator;
controlling (S114), by the light indicator controller, an illumination of the electrode light indicator and the cable light indicator based on the operational mode of the defibrillator,
wherein the controlling further includes:
controlling (S116), by the light indicator controller, a standby/startup illumination of the electrode light indicator and the cable light indicator representative of at least one of a deactivation or an activation of the defibrillator when electrode is coupled to the defibrillator by cable;
controlling (S118), by the light indicator controller, an application illumination of the electrode light indicator and the cable light indicator representative of a detection, by the defibrillator, of the electrode being applied to a patient;
controlling (S120), by the light indicator controller, a therapy alert illumination of the electrode light indicator and the cable light indicator representative of at least one of a heart rhythm analysis or a shock delivery being executed by the defibrillator; and
controlling (S126), by the light indicator controller, an intervention illumination of the electrode light indicator and the cable light indicator representative of an intervention monitoring being executed by the defibrillator.

## Patentansprüche

1. Eine Defibrillationseinheit (10), die Folgendes umfasst:
einen Defibrillator (20);
eine Elektrode (30), die eine Elektrodenleuchtanzeige (31) einschließt;
ein Kabel (50), ausgebildet, um die Elektrode mit dem Defibrillator zu verbinden, wobei das Kabel eine Kabelleuchtanzeige (51) einschließt; und
eine Leuchtanzeigensteuerung (70) zur Steuerung einer Anzeige eines Funktionsstatus der Defibrillationseinheit (10), wobei die Leuchtanzeigensteuerung (70) konfiguriert ist, um
einen Betriebszustand des Defibrillators zu bestimmen;
eine Beleuchtung der Elektrodenleuchtanzeige und der Kabelleuchtanzeige anhand des Betriebszustands des Defibrillators zu steuern,
wobei
die Leuchtanzeigensteuerung weiter konfiguriert ist, um Folgendes zu steuern:
eine Bereitschafts-/Einschaltbeleuchtung der Elektrodenleuchtanzeige und der Kabelleuchtanzeige, die eine Deaktivierung und/oder eine Aktivierung des Defibrillators anzeigen, wenn die Elektrode mit dem Defibrillator über das Kabel gekoppelt ist;
eine Auftragsbeleuchtung der Elektrodenleuchtanzeige und der Kabelleuchtanzeige, die eine Erkennung durch den Defibrillator anzeigen, dass die Elektrode an einen Patienten angebracht wird;
eine Therapie-Benachrichtigungsbeleuchtung der Elektrodenleuchtanzeige und der Kabelleuchtanzeige, die eine Herzrhythmusanalyse und/oder eine Schockabgabe anzeigen, die von dem Defibrillator durchgeführt werden;
eine Interventionsüberwachung der Elektrodenleuchtanzeige und der Kabelleuchtanzeige, die eine Interventionsüberwachung anzeigt, die von dem Defibrillator durchgeführt wird; und,
basierend auf dem Betriebszustand des Defibrillators, eine Beleuchtung mindestens eines von Folgendem: Aufreißlaschen an den Elektroden, einer CPR-Feedbackscheibe oder einem Elektrodenköcher oder einer Elektrodenkassette.

2. Eine Leuchtanzeigensteuerung (70) zur Steuerung einer Anzeige eines Funktionsstatus einer Defibrillationseinheit (10), die einen Defibrillator (20), eine Elektrode (30) mit einer Elektrodenleuchtanzeige (31) und ein Kabel (50) mit einer Kabelleuchtanzeige (51) einschließt, wobei die Leuchtanzeigensteuerung (70) Folgendes umfasst:
ein nicht flüchtiges maschinenlesbares Speichermedium, codiert mit Anweisungen zur Ausführung durch mindestens einen Prozessor, wobei das nicht flüchtige maschinenlesbare Speichermedium die Anweisungen einschließt, um
den Funktionsstatus eines Defibrillators zu bestimmen;
eine Beleuchtung der Elektrodenleuchtanzeige und der Kabelleuchtanzeige anhand des Betriebszustands des Defibrillators zu steuern;
wobei die Anweisungen weiter Folgendes einschließen:
das Steuern einer Bereitschafts-/Einschaltbeleuchtung der Elektrodenleuchtanzeige und der Kabelleuchtanzeige, die eine Deaktivierung und/oder eine Aktivierung des Defibrillators anzeigen, wenn die Elektrode mit dem Defibrillator über das Kabel gekoppelt ist;
das Steuern einer Auftragsbeleuchtung der Elektrodenleuchtanzeige und der Kabelleuchtanzeige, die eine Erkennung durch den Defibrillator anzeigen, dass die Elektrode an einen Patienten angebracht wird;
das Steuern einer Therapie-Benachrichtigungsbeleuchtung der Elektrodenleuchtanzeige und der Kabelleuchtanzeige, die eine Herzrhythmusanalyse und/oder eine Schockabgabe anzeigen, die von dem Defibrillator durchgeführt werden;
das Steuern einer Interventionsüberwachung der Elektrodenleuchtanzeige und der Kabelleuchtanzeige, die eine Interventionsüberwachung anzeigt, die von dem Defibrillator durchgeführt wird; und,
das Steuern, basierend auf dem Betriebszustand des Defibrillators, einer Beleuchtung mindestens eines von Folgendem: Aufreißlaschen an den Elektroden, einer CPR-Feedbackscheibe oder einem Elektrodenköcher oder einer Elektrodenkassette.

3. Ein Verfahren (110), ausführbar von einer Leuchtanzeigensteuerung (70) zur Steuerung einer Anzeige eines Funktionsstatus einer Defibrillationseinheit (10), die einen Defibrillator (20), eine Elektrode (30) mit einer Elektrodenleuchtanzeige (31) und ein Kabel (50) mit einer Kabelleuchtanzeige (51) einschließt, wobei das Verfahren Folgendes umfasst:
das Bestimmen eines Betriebszustands des Defibrillators durch die Leuchtanzeigensteuerung;
das Steuern (S114), durch die Leuchtanzeigensteuerung, einer Beleuchtung der Elektrodenleuchtanzeige und der Kabelleuchtanzeige anhand des Betriebszustands des Defibrillators,
wobei das Steuern weiter Folgendes einschließt:
das Steuern (S116), durch die Leuchtanzeigensteuerung, einer Bereitschafts-/Einschaltbeleuchtung der Elektrodenleuchtanzeige und der Kabelleuchtanzeige, die eine Deaktivierung und/oder eine Aktivierung des Defibrillators anzeigen, wenn die Elektrode mit dem Defibrillator über das Kabel gekoppelt ist;
das Steuern (S118), durch die Leuchtanzeigensteuerung, einer Auftragsbeleuchtung der Elektrodenleuchtanzeige und der Kabelleuchtanzeige, die eine Erkennung durch den Defibrillator anzeigen, dass die Elektrode an einen Patienten angebracht wird;
das Steuern (S120), durch die Leuchtanzeigensteuerung, einer Therapie-Benachrichtigungsbeleuchtung der Elektrodenleuchtanzeige und der Kabelleuchtanzeige, die eine Herzrhythmusanalyse und/oder eine Schockabgabe anzeigen, die von dem Defibrillator durchgeführt werden; und
das Steuern (S126), durch die Leuchtanzeigensteuerung, einer Interventionsüberwachung der Elektrodenleuchtanzeige und der Kabelleuchtanzeige, die eine Interventionsüberwachung anzeigt, die von dem Defibrillator durchgeführt wird.

## Revendications

1. Unité de défibrillation (10), comprenant :
un défibrillateur (20) ;
une électrode (30) comportant un indicateur lumineux d'électrode (31) ;
un câble (50) configuré pour relier l'électrode au défibrillateur, où le câble comporte un indicateur lumineux de câble (51) ; et un contrôleur d'indicateur lumineux (70) destiné à contrôler l'indication de l'état de fonctionnement de l'unité de défibrillation (10), où le contrôleur d'indicateur lumineux (70) est configuré pour :
déterminer un mode de fonctionnement du défibrillateur ;
commander un allumage de l'indicateur lumineux de l'électrode et d'indicateur lumineux du câble en fonction du mode de fonctionnement du défibrillateur,
où
le contrôleur d'indicateur lumineux est en outre configuré pour commander :
un allumage en mode veille/démarrage de l'indicateur lumineux de l'électrode et de l'indicateur lumineux du câble, indiquant au moins l'une des situations suivantes : une désactivation ou une activation du défibrillateur lorsque l'électrode est reliée au défibrillateur par le câble ;
un allumage d'application de l'indicateur lumineux d'électrode et de l'indicateur lumineux du câble, indiquant que le défibrillateur a détecté que l'électrode est appliquée sur un patient ;
un allumage d'alerte thérapeutique de l'indicateurs lumineux d'électrode et de l'indicateur lumineux du câble, indiquant qu'au moins l'une des opérations suivantes est en cours : une analyse du rythme cardiaque ou la délivrance d'un choc par le défibrillateur ;
une allumage d'intervention de l'indicateur lumineux de l'électrode et de l'indicateur lumineux du câble, indiquant qu'une surveillance d'intervention est en cours d'exécution par le défibrillateur ; et,
en fonction du mode de fonctionnement du défibrillateur, un allumage d'au moins l'un des éléments suivants : les languettes de retrait sur les électrodes, un disque de retour d'information RCP, ou un étui ou une cartouche d'électrodes.

2. Contrôleur d'indicateurs lumineux (70) destiné à contrôler l'indication d'un état de fonctionnement d'une unité de défibrillation (10) comprenant un défibrillateur (20), une électrode (30) dotée d'un indicateur lumineux d'électrode (31) et un câble (50) doté d'un indicateur lumineux de câble (51), le contrôleur d'indicateurs lumineux (70) comprenant :
un support de stockage non volatile lisible par machine, sur lequel sont codées des instructions destinées à être exécutées par au moins un processeur, ledit support de stockage non volatile lisible par machine comprenant les instructions pour:
déterminer l'état de fonctionnement d'un défibrillateur ;
commander un allumage de l'indicateur lumineux d'électrode et de l'indicateur lumineux de câble en fonction du mode de fonctionnement du défibrillateur,
où lesdites instructions comprenant en outre :
commander un allumage en mode veille/démarrage de l'indicateur lumineux d'électrode et de l'indicateur lumineux de câble, représentatif d'au moins l'une parmi une désactivation ou une activation du défibrillateur lorsque l'électrode est couplée au défibrillateur par le câble ;
commander un allumage d'application de l'indicateur lumineux d'électrode et de l'indicateur lumineux de câble, indiquant que le défibrillateur a détecté que l'électrode est appliquée sur un patient ;
commander un allumage d'alerte thérapeutique de l'indicateur lumineux d'électrode et de l'indicateur lumineux de câble, indiquant qu'au moins l'une des opérations suivantes est en cours : une analyse du rythme cardiaque ou la délivrance d'un choc par le défibrillateur ;
commander un allumage d'intervention de l'indicateur lumineux de l'électrode et de l'indicateur lumineux du câble, indiquant qu'une surveillance d'intervention est en cours d'exécution par le défibrillateur ; et,
commander, en fonction du mode de fonctionnement du défibrillateur, un allumage d'au moins l'un des éléments suivants : les languettes de retrait sur les électrodes, un disque de retour d'information RCP, ou un étui ou une cartouche d'électrodes.

3. Procédé (110) pouvant être exécuté par un contrôleur d'indicateurs lumineux (70) pour contrôler l'indication d'un état de fonctionnement d'une unité de défibrillation (10) comprenant un défibrillateur (20), une électrode (30) dotée d'un indicateur lumineux d'électrode (31) et un câble (50) doté d'un indicateur lumineux de câble (51), le procédé comprenant :
la détermination, par le contrôleur d'indicateurs lumineux, d'un mode de fonctionnement du défibrillateur ;
la commande (S114), par le contrôleur d'indicateurs lumineux, de l'allumage de l'indicateur lumineux d'électrode et de l'indicateur lumineux de câble en fonction du mode de fonctionnement du défibrillateur,
où la commande comprend en outre :
la commande (S116), par le contrôleur d'indicateurs lumineux, d'un allumage en veille/démarrage de l'indicateur lumineux d'électrode et de l'indicateur lumineux de câble, représentatif d'au moins l'une parmi une désactivation ou une activation du défibrillateur lorsque l'électrode est couplée au défibrillateur par le câble ;
la commande (S118), par le contrôleur d'indicateurs lumineux, d'un allumage d'application de l'indicateur lumineux d'électrode et de l'indicateur lumineux de câble, indiquant que le défibrillateur a détecté que l'électrode est appliquée sur un patient ;
la commande (S120), par le contrôleur d'indicateurs lumineux, d'un allumage d'alerte thérapeutique de l'indicateur lumineux d'électrode et de l'indicateur lumineux de câble, indiquant qu'au moins l'une des opérations suivantes est en cours : une analyse du rythme cardiaque ou la délivrance d'un choc par le défibrillateur ; et
la commande (S126), par le contrôleur d'indicateurs lumineux, d'un allumage d'intervention de l'indicateur lumineux de l'électrode et de l'indicateur lumineux du câble, indiquant qu'une surveillance d'intervention est en cours d'exécution par le défibrillateur.
